# EUROPEAN PATENT APPLICATION

(11) **EP 1 354 960 A1**
(43) Date of publication of application: **22.10.2003**
(21) Application number: 02007819.2
(22) Date of filing: 08.04.2002
(51) Int. Cl.: C12Q 1/00

(54) **Assay for synthesis activity of hydrolases**

(71) Applicant: Degussa AG, 40474 Düsseldorf (DE)
(72) Inventor: Bornscheuer, Uwe T., Prof. Dr., 17489 Greifswald (DE); Kanarzycka, Monika, 17489 Greifswald (DE); Hills, Geoffrey, Dr., 45355 Essen (DE)

(57) **Abstract**

The present invention provides an assay for testing hydrolase enzymes, in particular esterases or lipases, for their ability to catalyse synthesis reactions in nonaqueous, organic media. The assay can serve as a tool for high-throughput screening of libraries of enzymes stemming e.g. from directed evolution processes.

The assay comprises an enzymatically catalysed transesterification reaction releasing a carbonyl compound indirectly measured by its reaction with a further molecule thus rendering it detectable.

## Description

The present invention concerns a way to detect the feasibility of a special family of enzymes to produce chemical compounds. In particular the invention is directed to an assay and a method to screen libraries of hydrolases, in particular esterases, lipases, proteases, amidases and acylases for their facility to catalyse esterification, transesterification and interesterification reactions in non-aqueous, organic media.

Hydrolases, in particular lipases and esterases are versatile biocatalysts and find increasing application in organic syntheses (U.T. Bornscheuer, R. J. Kazlauskas, Hydrolases in organic synthesis - regio- and stereoselective biotransformations, Wiley-VCH, Weinheim, 1999; K. Faber, Biotransformations in Organic Chemistry, 4 ed., Springer, Berlin, 2000, K.Drauz, H. Waldmann (Ed.), Enzymes in Organic Synthesis, Wiley-VCH, Weinheim, 2002) The main interest stems for their good to excellent stereoselectivity and their exceptionally high activity and stability in organic systems. Thus, not only hydrolysis of esters can be studied, but also synthesis reactions are possible. Indeed, the majority of reactions are currently performed in organic media and are even performed on industrial scale (A. Liese et al., Industrial Biotransformations, Wiley-VCH, Weinheim, 2000; U. T. Bornscheuer, in Biotechnology, Vol. 8b (Eds.: H. J. Rehm, G. Reed, A. Pühler, P. J. W. Stadler, D. R. Kelly), Wiley-VCH, Weinheim, 2000, pp. 277-294; F. Balkenhohl et al., J. Prakt. Chem. 1997, 339, 381-384). This includes the synthesis of optically pure building blocks, flavors and fragances, lipid modification and also synthesis of simple esters such as myristyl myristate for cosmetic applications.

Identification of suitable biocatalysts is usually performed by small-scale esterification reactions, which can be monitored by GC or HPLC analysis to determine conversion and - if applicable - optically purities. Broad investigations of a large number of enzymes - especially of huge mutant libraries accessible by directed evolution (F. H. Arnold, A. A. Volkov, Curr. Opin. Chem. Biol. 1999, 3, 54-59; F. H. Arnold, Acc. Chem. Res. 1998, 31, 125-131; U.T. Bornscheuer, Angew. Chem. Int. Ed. Engl. 1998, 37, 3105-3108; M.T. Reetz, K.-E. Jaeger, Topic Curr. Chem. 1999, 200, 31-57) - and optimisation of reaction conditions (different alcohols or acyl donors, solvents, temperature variation etc.) are therefore very time-consuming.

Alternatively, active (and stereoselective) biocatalysts are usually identified by means of hydrolytic assays, for which a considerable number of useful high-throughput methods have been developed recently (M. T. Reetz, Angew. Chem. Int. Ed. Engl. 2001, 40, 284-310; D. Wahler et al., Angew. Chem. Int. Ed. Engl. 2001, 40, 4457-4460; D. Wahler, J.L. Reymond, Curr. Opin. Biotechnol. 2001 12, 535-544; M. Baumann et al., Angew. Chem. Int. Ed. Engl. 2001, 40, 4201-4204; L. E. Janes, R. J. Kazlauskas, J. Org. Chem. 1997, 62, 4560-4561). Unfortunately, the above mentioned optimisations can not be studied in aqueous systems if a reaction in organic solvents is envisaged and it often turns out that results from hydrolysis reactions can not be transferred to synthesis in organic solvents or solvent-free systems.

The object of the present invention was, therefore, to find an assay which allows the probing of large numbers of hydrolases according to their applicability in catalysing esterification, transesterification and interesterification reactions in non-aqueous, organic systems on a high-throughput level.

By applying an one pot assay to detect the synthesis activity of hydrolases comprising:
(i) transesterification of an ester of a carboxylic acid and a vinylogous alcohol with a further alcohol or ester in the presence or absence of a non aqueous solvent and the respective enzyme,
(ii) reacting the thus produced carbonylic compound with a further molecule indifferent to the reaction under (i) whereby
(iii) an easily detectable reaction product is obtained, the artisan gains the possibility to manufacture a huge amount of analysis of such synthesis reactions within a short time period automatically and gets information not only on qualitative but also on quantitative turnovers helping to select the most appropriate enzymes and/or reaction conditions and/or substrates for the transformation in question.

As already mentioned the screening can be done automatically by robots or the like. Vessels in which hundreds of reactions can be performed and analysed in parallel are known to the skilled worker. Suitable are so called microtiter plates (MTPs) for such purpose. Also feasible is the use of other equipment known to the skilled worker for enzymatic reactions such as cuvettes, eppendorf tubes, or even agar plates.

Hydrolases are a separate class of enzymes (E.C. 3). They possess the possibility to cleave chemical bonds hydrolytically. They encompass subclasses of enzymes like esterases, lipases, proteases, amidases or acylases which also are advantageously screened by the assay of the invention.

It is a key feature of the present assay that during said transesterification reaction a carbonylic compound as reactive intermediate is produced which can be detected indirectly. The reactive intermediate has to fulfil special requirements to be advantageously applied in this assay. Firstly, it has to be released by the reaction of the hydrolase in question with the enol ester. Secondly, it shall react with a further molecule present, thus rendering the characteristics of this compound in a way to be easily detectable. Alcohols matching these requirements are those which can be released irreversibly from the acid moiety like vinylogous alcohols having a C=C-double adjacent to the alcoholic function. Upon release such alcohols undergo a keto-enol tautomerisation yielding the corresponding carbonyl compound.
The skilled worker is aware of different kinds of reagents capable of reacting in proposed ways. Most preferably vinyl alcohol or 1-propen-2-ol leading to acetaldehyde or acetone, respectively, are engaged.

The carbonylic compound, like acetaldehyde or acetone, as already mentioned in turn reacts with a further molecule rendering it easily detectable.
According to the invention the skilled worker is well aware of different kinds of easy detection methods. These have to be applied in accordance with the detectable reaction product to be measured. Appropriate methods include e.g. spectrophotometric and fluorimetric measurements (D. Wahler et al., Angew. Chem. Int. Ed. Engl. 2001, 40, 4457-4460; E. Henke, U. T. Bornscheuer, Biol. Chem. 1999, 380, 1029-1033; M. Baumann et al., Angew. Chem. Int. Ed. Engl. 2001, 40, 4201-4204; L. E. Janes, R. J. Kazlauskas, J. Org. Chem. 1997, 62, 4560-4561). Preferred are detection methods which can provide information both on qualitative and on quantitative transesterification progress. More preferred are those which are easy to handle and best implemented in automatic processing. Spectrometric detection serves well in this respect. Most preferred a fluorimetric analysis method is applied.
The further molecule to be modified has to have special features to be successfully applied in the assay of the invention. On the one hand it has to act indifferent in view of the enzymatic transesterification, neither inhibiting nor otherwise adversely effecting it. One the other hand it shall react easily and quantitatively with the carbonylic compound released by the enzymatic transesterification under the conditions employed.
Moreover, relative to the detection method applied the further molecule's characteristics are thereby changed in such a way as to be monitored with an appropriate sensitivity. Thus the selection of the further molecule is directly linked to the carbonyl compound produced and the detection method employed. Numerous reagents are known for the derivatisation of carbonyl compounds (for an overview see M. Vogel et al., Fresenius J. Anal. Chem. 2000, 366, 781-791) and are used mostly in e.g. environmental analysis. However, the derivatisation conditions used may be incompatible with enzymatic reactions and the derivative of the carbonyl compound must be separated (e.g. by HPLC) from the derivatisation reagent as their mixture can not be analyzed directly. The skilled artisan may think of special further molecules like dansyl derivatives (S. Houdier et al., Anal. Chim. Acta 1999, 382, 253-263) or perylenes (H. Langhals, W. Jona, Chem. Eur. J. 1998, 4, 2110-2116). Advantageously, in view of the release of a carbonylic compound, like acetaldehyde or acetone, the indifferent reagent is selected from the group of aromatic hydrazines. Most preferred the reagent 4-(7-nitro-2,1,3-benzoxadiazole)-hydrazine (or the derivatives as mentioned in ref. S. Uzu et al, Analyst 1990, 115, 1477-1482) is employed. The commercially available 4-(7-nitro-2,1,3-benzoxadiazole)-hydrazine (NBD-H) is a promising reagent in this respect, as it shows almost no fluorescence, whereas the corresponding hydrazone shows very high fluorescence. Moreover, the derivatisation can be performed in organic solvents at variable temperatures.

The hydrolases to be screened shall be employed for esterification, transesterification and interesterification reactions on large scale production. Inherently to these reactions is the fact that they can not be performed in aqueous solution. As already mentioned hydrolases tested for hydrolysis of esters in aqueous medium are seldom thus effective if employed in non aqueous systems. Therefore, the assay according to the invention is advantageously also performed in non-aqueous solution or without the addition of a further solvent, e.g. in neat form or in the alcohol to be transformed. Non-aqueous solvents which may be used shall have the ability to dissolve all and may not disturb the reactions between the reactants employed. Moreover, feasible media encompass those not adversely effecting the detection method applied. Preferably the solvents in which the assay is conducted are selected from the group of alkanes, cycloalkanes, aromatic hydrocarbons, ethers, tertiary alcohols or other solvents known to the artisan used as organic media in enzymatic synthesis reactions (U.T. Bornscheuer, R. J. Kazlauskas, Hydrolases in organic synthesis - regio- and stereoselective biotransformations, Wiley-VCH, Weinheim, 1999; K. Faber, Biotransformations in Organic Chemistry, 4 ed., Springer, Berlin, 2000, K.Drauz, H. Waldmann (Ed.), Enzymes in Organic Synthesis, Wiley-VCH, Weinheim, 2002). Most preferred are linear or branched pentane, hexane, heptane, toluene, octane, cyclopentane, cyclohexane, methyl cyclohexane, petrol ether, methyl-t-butyl ether, diethyl ether, diisopropylether, tertiary butanol, 2-methyl-2-butanol, DMF, DMSO, THF, methylenchloride, chloroform or mixtures thereof.

The assay according to the invention is run most properly at a temperature where the hydrolase tested are acting favourably. Thus the temperature ranges from -15°C to over 100°C for, for example, enzymes of thermophilic organisms. In usual cases the temperature applied varies from 0°C to 60°C, most preferred from 15°C to 50°C.

In a further embodiment the invention is directed to a method for detecting the synthesis activity of hydrolases using an assay according to the invention. Furthermore, the substrate specificity and stereoselectivity of a particular hydrolase enzyme or enzymes can be efficiently determined by varying the carboxylic acid and alcohol moieties of the compounds to be tested in the assay.

The assay format is in principle also applicable to the determination of enantioselectivities of special substrates. In case of chiral carboxylic acids, the corresponding vinyl esters can be used. These compounds can be easily synthesized from the chiral carboxylic acid e.g. using vinyl acetate in the presence of palladium(II)acetate (E. Henke et al., Chem. Month. 2000, 131, 633-638).
For the resolution of e.g. chiral alcohols, vinyl acetate can serve as acyl donor.

One further aspect of the assay in question is its use as a probe for special carboxylic acids or alcohols in organic media if specifically acting enzymes are employed.

For an example the new assay format based on hydrolase catalysed transesterifications between vinyl laurate and 1-propanol is described. Acetaldehyde released stoichiometrically reacts *in situ* with a hydrazine producing a fluorogenic derivative, which can be quantified by fluorescence measurements (Scheme 1).

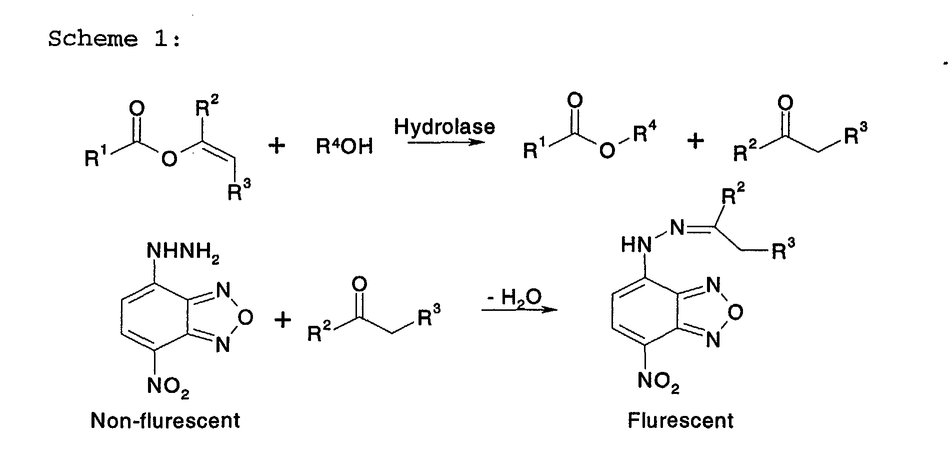

R¹ may be the rest of the carboxylic acid as defined in the text or (C₁-C₈)-Alkyl, (C₂-C₈)-Alkoxyalkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl- (C₆-C₁₈)-Aryl, (C₁-C₈)-Alkyl- (C₃-C₁₈)-Heteroaryl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl.

R² or R³ are independently of each other H or (C₁-C₈)-Alkyl, (C₂-C₈)-Alkoxyalkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl- (C₃-C₁₈)-Heteroaryl, (C₃-C₈)-Cycloalkyl, (C₁-C₈)-Alkyl- (C₃-C₈)-Cycloalkyl, (C₃-C₈)-Cycloalkyl-(C₁-C₈)-Alkyl.

R⁴ is (C₁-C₈)-Alkyl, (C₂-C₈)-Alkoxyalkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₈)-Heteroaryl, (C₃-C₈)-Cycloalkyl, (C₁-C₈)-Alkyl-(C₃-C₈)-Cycloalkyl, (C₃-C₈)-Cycloalkyl- (C₁-C₈)-Alkyl.

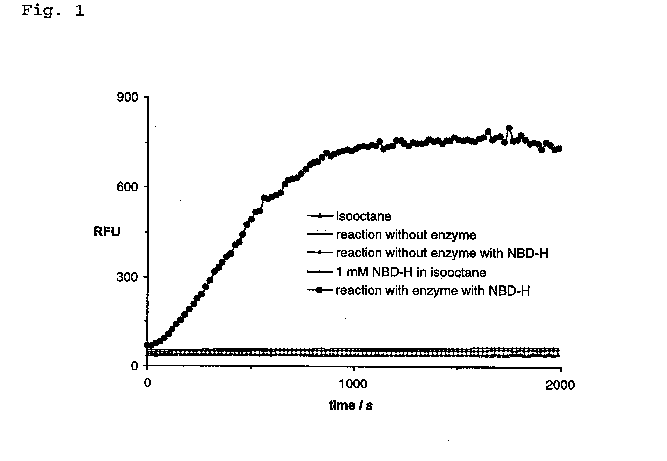

This model reaction catalysed by lipase B from *Candida antarctica* (CAL-B) was studied in greater detail. As a control, the reaction progress was monitored by GC-analysis revealing that >93% conversion can be achieved within ∼45 min in isooctane or n-hexane (at 1:1 ratio of substrates using 0.5 % (w/w) CAL-B). Next, the model reaction was performed in microtiter plates (MTPs) in the presence of NBD-H. Fig. 1 shows a typical time course of this reaction as monitored by fluorescence measurements. This clearly shows, that the progress of the reaction can be easily monitored. However, it is recommended to use tape-sealed MTPs to avoid evaporation of the highly volatile acetaldehyde, here measurement of fluorescence is preferentially performed from the bottom of the MTP. Moreover, in most cases a good correlation between increase in fluorescence and conversion determined by GC analysis from samples of the reaction mixture can be found. Thus, this assay format is extremely versatile for the determination of synthetic activity of hydrolases in a high-throughput format and thus enables a rapid identification of active enzymes or appropriate reaction conditions.

The variety of carboxylic acids which can be applied in the synthesis screening assay is broad. Examples are acetic acid, propionic acid etc. , acrylic acid, fatty acids like oleic acid, aromatic acids like benzoic acid, lactic acid, natural and unnatural amino acids, cinnamic acid, 2-methylbutanoic acid, 2-ethylhexanoic acid, 2-chloropropionic acid or sugar acids.

As alcohol compounds like n-alcohols, 2-ethylhexanol, 2-alcohols such as isopropanol or 2-butanol, diols such as 1,2-butandiol, polyols such as carbohydrates or glycerin or polyglycerin, polyethers such as polyethyleneglycol, phenol, halogen containing alcohols such as 2-chloroethanol, 2-chloropropanol can be used.

The compounds listed are by no means intended to be restrictive to the invention. Principally, all types of carboxylic acids and alcohols are possible within the focussed limitations.

(C₁-C₈)-Alkyl may be regarded as being methyl, ethyl, n-propyl, isopropyl, n-butyl, isobutyl, sec-butyl, tert-butyl, pentyl, hexyl, heptyl or octyl, including all isomers either saturated or unsaturated. They may be mono-or poly-substituted by (C₁-C₈)-alkoxy, (C₁-C₈)-haloalkyl, OH, halogen, NH₂, NO₂, SH, S-(C₁-C₈)-alkyl, (C₁-C₈)-acyl. The radicals just described may be mono- or poly-substituted by halogens and/or by radicals containing N, O, P, S, Si atoms. They are in particular alkyl radicals of the type mentioned above that contain one or more of those hetero atoms in their chain or that are bonded to the molecule *via* one of those hetero atoms.

(C₃-C₈)-Cycloalkyl is to be understood as being cyclopropyl, cyclobutyl, cyclopentyl, cyclohexyl and cycloheptyl radicals, etc. either saturated or unsaturated. They may be substituted by one or more halogens and/or radicals containing an N, O, P, S atom and/or may have in the ring radicals containing an N, O, P, S atom, such as, for example, 1-, 2-, 3-, 4-piperidyl, 1-, 2-, 3-pyrrolidinyl, 2-, 3-tetrahydrofuryl, 2-, 3-, 4-morpholinyl. They may also be mono- or poly-substituted by (C₁-C₈)-alkoxy, (C₁-C₈)-haloalkyl, OH, Cl, NH₂, NO₂, (C₁-C₈)-acyl.

A (C₆-C₁₈)-aryl radical is to be understood as being an aromatic radical having from 6 to 18 carbon atoms. Such radicals include especially compounds such as phenyl, naphthyl, anthryl, phenanthryl, biphenyl radicals. It may be mono- or poly-substituted by (C₁-C₈)-alkoxy, (C₁-C₈)-haloalkyl, OH, halogen, NH₂, NO₂, SH, S-(C₁-C₈)-alkyl, (C₁-C₈)-acyl .

A (C₇-C₁₉)-aralkyl radical is a (C₆-C₁₈)-aryl radical bonded to the molecule *via* a (C₁-C₈)-alkyl radical.

(C₁-C₈)-Alkoxy is a (C₁-C₈)-alkyl radical bonded to the molecule in question *via* an oxygen atom.

(C₁-C₈)-Haloalkyl is a (C₁-C₈)-alkyl radical substituted by one or more halogen atoms.

Within the scope of the invention, a (C₃-C₁₈)-heteroaryl radical denotes a five-, six- or seven-membered aromatic ring system of from 3 to 18 carbon atoms that contains hetero atoms such as, for example, nitrogen, oxygen or sulfur in the ring. Such heteroaromatic radicals are to be regarded as being especially radicals such as 1-, 2-, 3-furyl, such as 1-, 2-, 3-pyrrolyl, 1-, 2-, 3-thienyl, 2-, 3-, 4-pyridyl, 2-, 3-, 4-, 5-, 6-, 7-indolyl, 3-, 4-, 5-pyrazolyl, 2-, 4-, 5-imidazolyl, acridinyl, quinolinyl, phenanthridinyl, 2-, 4-, 5-, 6-pyrimidinyl. It may be mono-or poly-substituted by (C₁-C₈)-alkoxy, (C₁-C₈)-haloalkyl, OH, halogen, NH₂, NO₂, SH, S-(C₁-C₈)-alkyl, (C₁-C₈)-acyl.

A (C₄-C₁₉)-heteroaralkyl is to be understood as being a heteroaromatic system corresponding to the (C₇-C₁₉)-aralkyl radical.

(C₁-C₈)-Acyl is to be understood as being a (C₁-C₈)-alkyl radical bonded to the molecule *via* a -C=O- function.

Suitable halogens are fluorine, chlorine, bromine and iodine.

Within the scope of the invention, the expression enantiomerically concentrated is to be understood as meaning the proportion of an enantiomer in admixture with its optical antipodes in a range > 50 % and < 100 %.

### Experimental

Materials: 4-hydrazino-7-nitro-2,1,3-benzoxadiazole (NBD-H) was purchased from Fluka. All other chemicals were obtained at the highest purity available from common commercial suppliers.

Fluorimetric analysis was performed with the FLUOstar *Galaxy* from BMG Labtechnologies GmbH (Offenburg, Germany) using an excitation wave-length of 485 nm, and an emission wave-length of 520 nm or 590 nm at gain 0. The total reaction volume was 200-220 µl per well.

Gas-chromatographic analysis: This was performed on a Hewlett Packard GC (5890 Series II with HP 3365 Series II ChemStation) equipped with a Permabond FFAP column (Macherey & Nagel, Düren, Germany), 25 m x 0.25 mm. Samples were analysed under the following conditions: isothermal (140°C), detection temperature: 250°C, injection temperature: 230°C. Hydrogen served as carrier gas.

Lipase-catalyzed synthesis of 1-propyl laurate: Solvent free system, 1:1 ratio (vinyl laurate:1-propanol): 260 µl (1 mmol) of vinyl laurate and 60 µl (1 mmol) of 1-propanol were combined in an eppendorf tube (1.5 ml) and CAL-B (1-10 mg) was added. The mixture was shaken at 900 rpm at 60°C. Samples were diluted with chloroform, vortexted, centrifuged and analyzed by GC. In a similar manner, reactions at a 1:10 ratio were performed. Reactions with solvents: 77,5 µl (0.3 mmol) of vinyl laurate, 22,5 µl (0.3 mmol) of 1-propanol and 900 µl of solvent (n-hexane, isooctane, petroleum ether) are combined in an eppendorf tube (1.5 ml). Reactions were performed and analyzed as described above.

Fluorescence of NBD-H: In general, 200 µl of a solution of NBD-H (0.1, 0.25, 0.5 and 1 mM) in various solvents (methanol, 1-propanol, *n*-hexane, isooctane, petrol ether (boiling range 100-140°C), DMSO) were transferred into a microtiter plate. Then fluorescence was measured at 20°C, 25°C or 45°C for 30-50 min at the wave-lengths indicated above. In a similar manner, derivatization of acetaldehyde was studied by addition of 10 µl acetaldehyde (0.1 nmol) dissolved in the organic solvent using a micro pump within the fluorimeter. Reaction mixtures were mixed with the internal shaker of the fluorimeter at 170 rpm.

General procedure for the determination of synthetic activity in a microtiter plate: A mixture (210 µl) of vinyl laurate (200 nmol), 1-propanol (2 µmol) (1:10 ratio) and NBD-H (200 nmol) in 210 µl isooctane was prepared. After addition of ∼0.02 mg CAL-B, the plates were tape-sealed and inserted into the fluorimeter and shaken at 45°C at 170 rpm. The change in fluorescence was monitored by measurement from the bottom for 20 min. As weighing of these small amounts of enzyme are very difficult to achieve reproducible results, a known amount of enzyme was dissolved in phosphate buffer and then distributed to the wells of a MTP. Next, the solution was freeze-dried to ensure an exact amount of biocatalysts per reaction. Finally, substrates, organic solvent and NDB-H were added as described above.

## Claims

1. Assay to detect the synthesis activity of hydrolases comprising:
(i) transesterification of an ester of a carboxylic acid and a vinylogous alcohol with a further alcohol or ester in non-aqueous media, in the presence or absence of a non-aqueous solvent, and the respective enzyme,
(ii) reacting the thus produced carbonylic compound with a further molecule indifferent to the reaction under (i) whereby
(iii) an easily detectable reaction product is obtained.

2. Assay according to claim 1,
**characterised by** that the hydrolases are esterases, lipases, proteases, amidases or acylases.

3. Assay according to claim 1 and/or 2,
**characterised in that**,
the α,β-unsatured moiety of the ester stems from vinyl alcohol or 1-propen-2-ol.

4. Assay according to any one of the preceding claims,
**characterised in that**,
the indifferent reagent is selected from the group of aromatic hydrazines.

5. Assay according to claim 4,
**characterised in that**,
the reagent is 4-(7-nitro-2,1,3-benzoxadiazole)-hydrazine.

6. Assay according to any one of the preceding claims,
**characterised in that**,
the organic solvent is selected from the group of alkanes or cycloalkanes, aromatic hydrocarbons, ethers, teriary alcohols.

7. Assay according to any one of the preceding claims,
**characterised in that**,
the temperature applied varies from -15°C to 100°C, preferably 0°C to 60°C, more preferably 15°C to 50°C.

8. Method for detecting the synthesis activity of hydrolases using a synthesis assay according to any one of the preceding claims.

9. Method for determining the substrate specificity of hydrolases using a synthesis assay according to any one of the preceding claims.

10. Method for determining the stereoselectivity of hydrolases using a synthesis assay according to any one of the preceding claims.

11. Method for determining an alcohol or carboxylic acid compound using a synthesis assay according to any one of the preceding claims in connection with a alcohol-or carboxylic acid-specific hydrolase.

12. Method for determining the enantioselectivity of chiral alcohols and chiral carboxylic acids using a synthesis assay according to any one of the preceding claims.
